# EUROPEAN PATENT APPLICATION

(11) **EP 3 586 725 A1**
(43) Date of publication of application: **01.01.2020**
(21) Application number: 18180548.2
(22) Date of filing: 28.06.2018
(51) Int. Cl.: A61B 5/00, A61B 5/021

(54) **BLOOD PRESSURE MEASUREMENT ANALYSIS METHOD AND SYSTEM**

(71) Applicant: Koninklijke Philips N.V., 5656 AE Eindhoven (NL)
(72) Inventor: SARTOR, Francesco, 5656 AE Eindhoven (NL); COX, Lieke, 5656 AE Eindhoven (NL); SHRUBSOLE, Paul Anthony, 5656 AE Eindhoven (NL)
(74) Representative: de Haan, Poul Erik

(57) **Abstract**

A method (10) for analyzing blood pressure measurements to determine any contextual or measurement tester related cause of the outlier. The method comprises identifying an obtained measurement as an outlier by comparing it with historical measurements for the subject. Any possible contextual cause of the outlier is first determined based on data in a database of contextual data for the subject. A database of historical measurement data for the tester who collected the measurement is then accessed and a determination made as to whether the outlier is attributable to the tester. Finally, the measurement, outlier status and any identified contextual or outlier-attributable cause are output. In embodiments, the measurement may be appended to a database, labelled as a true measurement and/or labelled with a cause for outlier status. Appending as a true measurement may be performed dependent on no determination being made of a contextual or tester-related cause of the outlier.

## Description

### FIELD OF THE INVENTION

This invention relates to a method and system for analyzing blood pressure measurements, to identify outliers.

### BACKGROUND OF THE INVENTION

Blood pressure is a widely measured vital sign of patients. It can be measured in a hospital or clinical environment, or alternatively at a patient's home using a remote monitoring system.

It is common for healthcare professionals to measure blood pressure inaccurately. Indeed, clinical research has shown that only a small percentage of healthcare professional staff are able to measure blood pressure accurately (Mohan et al. Office blood pressure measurement practices among community health provides (medical and paramedical) in northern district of India. Indian Health Journal. 2014.; Jones et al. Measuring blood pressure accurately: new and persist challenges. JAMA, 2003).

The most common errors made by staff include: applying the blood pressure cuff incorrectly, incorrect patient position, not measuring both arms, not measuring after appropriate rest, not measuring with the subject's arm at the right position, and not measuring in the absence of external stressors.

Inaccuracy in measuring blood pressure measurements can lead to incorrect diagnosis of hypertension. At the same time, wrongly dismissing unusual results can also be dangerous, leading to missing key indicators of serious medical conditions.

Means for reliably distinguishing between inaccurate measurements and outlier results indicative of genuine changes in patient condition are therefore generally required.

### SUMMARY OF THE INVENTION

The invention is defined by the claims.

According to examples in accordance with an aspect of the invention, there is provided a blood pressure measurement analysis method, comprising:
obtaining a blood pressure measurement for a subject, the measurement being associated with a known tester who performed the measurement;
comparing the measurement with historical measurements for the subject stored in a database, and determining based on this whether the obtained measurement is an outlier for the subject;
in the case the measurement is an outlier, accessing a database of contextual data for the subject, pertaining to contextual factors which have an effect on blood pressure, and determining based on this any contextual cause of the outlier measurement;
at least in the case of no contextual cause, accessing historical measurement data for the tester in a database, and determining based on this whether the outlier is attributable to the tester;
generating output information indicative of an outlier status of the measurement, and any determined contextual and/or tester attributed cause.

The invention hence makes use of databases of historical data for both the subject/patient and the tester who collects the blood pressure measurement, and contextual data for the subject to reliably derive whether an obtained measurement is an erroneous outlier or a true outlier measurement that should be actioned.

It has not previously been considered in particular to consult prior measurement data for the tester who collects the reading. By including this step, the chances of dismissing a genuine unusual result are greatly reduced, as it can readily be seen whether the tester has a history of collecting deviant results for example.

Using the particular combination of patient historical measurement data, contextual data and tester historical measurement data has not before been considered and provides for significantly improved medical outcomes for patients by permitting each outlier measurement to be reliably categorized or filtered in terms of its likely cause, thereby ensuring that appropriate clinical responses to unusual measurements can be put in action rapidly.

For example, embodiments of the invention permit rejecting of outlier measurements which are attributable to external factors (context or tester) and not health related factors. This thereby avoids inaccurate medical diagnoses which could lead either to unnecessary treatment, which can be deleterious to health, or lack of required treatment which can lead to escalation of genuine health issues.

The method itself in no embodiment ascribes any medical diagnostic attribution to an obtained measurement. Embodiments of the invention merely provide a tool for identifying certain possible non-medical causes for a measurement, with doctors or nurses then left to make any medical diagnostic judgments, these now being made on the basis of a more reliably accurate set of clinical data.

According to certain examples for instance, in the event that no contextual or tester-attributable cause is identified for an outlier measurement, the measurement may be exported to a measurement database, and labeled as a true measurement. Hence where no likely external cause is found, the method results in treating the measurement as genuine, allowing medical action to be taken with confidence that the measurement is not erroneous. Speed of medical response can thereby be increased, since doubt as to validity of the outlier measurement is reduced.

In some cases, the measurement may be exported, labelled as a true measurement only in the case that there is no contextual or tester-attributable cause of the measurement.

In either case, in the contrary case that a contextual or tester-attributable cause is identified for an outlier measurement, the method may comprise: exporting the measurement to a measurement database, labelled with the cause, or, alternatively, discarding the measurement.

Hence, where an external cause for the outlier measurement is identified, the measurement can be treated as a potentially erroneous measurement, allowing the medical response to be adapted accordingly. In particular, unnecessary treatment can be avoided in response to the measurement since it is either labelled (e.g. flagged) as a deviant measure with a likely external cause, or simply discarded so that the measurement is never taken into account in diagnostic decision making.

Discarding the measurement may comprise for example deleting the measurement and/or requesting a user repeat the measurement, or exporting the measurement to a separate database for erroneous measurements, such as an archive database. This permits in either case that clinical decisions are not made based on the measurement, by excluding the measurement from any database of genuine measurements which is used for clinical decision making.

According to any embodiment, the generated output information may comprise the measurement, labelled with the determined outlier status, and any determined contextual and/or tester attributed cause.

In some examples of the method, accessing the historical measurement data for the tester may be performed only in the case that no contextual cause is identified for the outlier. If a contextual cause is identified, the measurement can already be labelled as a likely erroneous measurement, avoiding the need for further steps to check for possible tester-attributable causes. This can hence simplify the method and reduce processing complexity in cases where a contextual attribution for an outlier measurement has been found. However, in other examples, both checks (context and tester) may be performed whether a contextual cause is found or not. This option can increase the reliability that a discarded measurement does have external cause and is not a genuine measurement. For example, both a contextual and tester-related cause might be found, increasing the certainty of any judgement about the reliability of the measurement.

The historical measurement data for the tester can take different forms.

In an advantageous set of examples, the historical measurement data for the tester may comprise performance data, the performance data being indicative of a historical accuracy of measurements of the tester.

For example, the performance data may indicate a measure of an average error of measurements taken by the tester or a distribution of their error in each measurement taken. Error may mean the deviation of measurements from true measurements or measurements as taken by other testers.

For example, the measure of average error may be a measure of an average disparity between
measurements taken by the tester for various subjects and measurements for the same subjects taken by other testers and/or
measurements taken by the tester for different subjects and historical measurements for the same subjects.

Both examples provide a sophisticated indication of likely performance or accuracy of the tester in taking measurements. The measure of disparity in each case may for example may be a distance from the measurement and an average (e.g. median or mean) value of the distribution of the comparative measurements (either the measurements for a given subject by other testers, or the historical measurements of the past patients).

In the first case, a significant average deviation in measurements from those of other testers would indicate a systematic error in the measurement technique of the tester (compared to other testers). If such a systematic deviation is found, it can be assumed that the measurements of other testers are on average accurate. For example, if all or most testers exhibited error in their measurements, a systematic deviation from measurements taken by other testers for the same subjects would not be visible as the whole set of results would simply be randomly distributed.

In the second case, if a tester has systematically recorded results for multiple past patients which deviate from those past patients' historical measures, this provides an indication that the tester has some systematic error in their measurement technique, since this result would not normally be expected for a typical patient population if the tester were accurate.

Determining whether the obtained measurement is attributable to a tester may in some examples comprise determining whether the average error for the tester exceeds some defined threshold error. The attribution may be made where the threshold is exceeded.

In other case, determining whether the outlier is attributable to the tester may comprise determining a measure of disparity between the obtained measurement and the historical measurements for the subject, and comparing this with said measure(s) of average error for the tester.

This is a more sophisticated assessment and may provide more reliable conclusions. For example where the newly determined disparity for the obtained measurement matches, or is within some defined threshold of, the average disparity for the tester, it can be reliably concluded that the deviation in the new measurement is caused by the same systematic error of the tester as has been exhibited in the past.

If on the other hand, the disparity for the new measurement for example is significantly greater (or significantly lower) than the average disparity for the tester, it may be an indication that there is some other underlying cause for the outlier, as the degree of deviation does not seem in accordance with the systematic error previously shown. Hence, the chance of discarding genuine unusual results for a patient, and hence potentially delaying essential treatment, merely because the tester has a historical inaccuracy can be avoided. It is checked whether the deviation in the new measurement is or is not likely caused by the tester's poor measurement performance.

Again, the measure of disparity in this case may be a difference between the measurement and an average (e.g. median or mean) value of the distribution of historical measurements.

According to at least one set of embodiments, determining whether the obtained measurement is an outlier may comprise determining a measure of disparity between the obtained measurements and the historic measurements for the subject and determining whether this exceeds a defined disparity threshold.

Again, the measure of disparity in this case may be a difference between the measurement and an average (e.g. median or mean) value of the distribution of historical measurements.

In examples, the contextual information may include information indicative of one or more of: subject medical record, subject medications, date and time of obtained measurement, subject height, subject weight, subject activity levels, whether subject smokes, subject diet, subject stress levels, subject breathing rate.

The method may according to certain embodiments, comprise determining a probability that the obtained measurement is an outlier.

For example, determining whether the measurement is an outlier may comprise determining said probability. If the probability exceeds a defined probability threshold, it may be determined that the measurement is an outlier.

Additionally or alternatively, the derived probability may be output as part of the output information.

Examples in accordance with a further aspect of the invention provide a computer program comprising code means for implementing the method according to any of the embodiments or examples outlined above, described below or defined in any of the claims when said program is run on a computer.

Examples in accordance with a further aspect of the invention provide an analysis system, comprising:
a first dataset storing historical blood pressure measurements for one or more subjects;
a second dataset comprising historical measurement data relating to historical measurements performed by one or more testers; and
a processor, having access to said databases, and adapted to:
   obtain a blood pressure measurement for a subject, the measurement being associated with a known tester who performed the measurement,
   compare the measurement with historical measurements for the subject stored in said first dataset, and determining based on this whether the obtained measurement is an outlier for the subject,
   in the case the measurement is an outlier, access a further database of contextual data for the patient, pertaining to contextual factors which have an effect on blood pressure, and determine based on this any contextual cause of the outlier measurement,
   at least in the case of no contextual cause, access historical measurement data for the tester from said second database, and determine based on this whether the outlier is attributable to the tester; and
   generate output information indicative of an outlier status of the measurement, and any determined contextual and/or tester attributed cause.

All options described above pertaining to the method aspect of this invention can equivalently be applied to the system aspect of the invention, and in particular may be applied in various examples and embodiments to the steps performed by the processor of the system.

The blood pressure measurement may be obtained for example from a data store or alternatively from a coupled patient monitor or blood pressure sensor. The measurement may be input manually by a user in other examples.

The first and second datasets defined above may be comprised by or be separate databases, or they may be incorporated as part of a single database.

In one set of embodiments, the system further comprises a blood pressure sensing means, communicatively coupled to the processor, for providing said blood pressure measurement to the processor. Hence in this case the system effectively provides a complete blood pressure measurement system, including components for both measuring blood pressure and analyzing the measurements.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a block diagram of an example method according to one or more embodiments;
Fig. 2 schematically illustrates approaches for determining whether an outlier is attributable to a testing personnel;
Fig. 3 is a block flow diagram illustrating one example method according to one or more embodiments; and
Fig. 4 schematically depicts an example analysis system according to one or more embodiments.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for analyzing blood pressure measurements to determine any contextual or measurement tester related cause of the outlier. The method comprises identifying an obtained measurement as an outlier by comparing it with historical measurements for the subject. Any possible contextual cause of the outlier is first determined by accessing data in a database of contextual data for the subject. A database of historical measurement data for the tester who collected the measurement is then accessed and a determination made as to whether the outlier is attributable to the tester. Finally, the measurement, outlier status and any identified contextual or outlier-attributable cause are output. In embodiments, the measurement may be appended to a database, labelled as a true measurement and/or labelled with a cause for outlier status. Appending as a true measurement may be performed dependent on no determination being made of a contextual or tester-related cause of the outlier.

Nurses take blood pressure readings throughout the day with blood pressure (BP) monitors. In home-monitoring applications, patients may also take readings. However, there are errors in taking the readings. Healthcare professionals can make basic errors in monitoring BP such as mis-cuffing patients, incorrect patient position, not measuring in both arms, not measuring after appropriate rest, not measuring with the arm at the correct positon, and not ensuring to measure at times when the patient is absent any external stressors.

Embodiments of the present invention provide a method and system which can identify outlier measurements and determine based on historical data whether the outlier may be attributable to poor measuring performance of the person who is taking the measurement, or whether it may be caused by contextual factors. The method and system is therefore able to distinguish a real blood pressure change from a user-induced outlier and provide feedback to health care professional staff accordingly. Measurements can be filtered or labelled according to their identified cause, to prevent false measurements being used to inform clinical decision making. This improves patient health outcomes by preventing unnecessary treatment and increasing certainty of unusual measurements, so that required treatment can be administered faster.

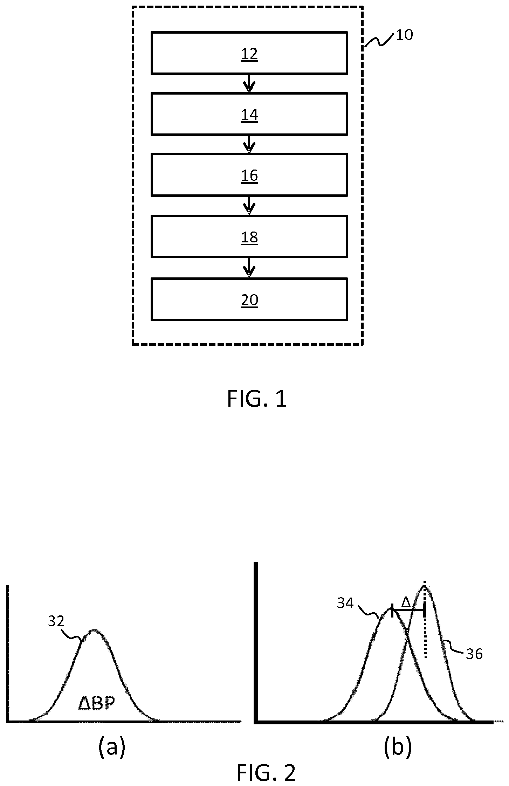

An example blood pressure measurement analysis method according to one or more embodiments is outlined in block diagram form in Fig. 1.

The method comprises a first step of obtaining 12 a blood pressure measurement for a subject, the measurement being associated with a known tester who performed the measurement. In some examples, the blood pressure measurement may be obtained from a data store in which collected data has been (e.g. temporarily) stored. In other examples, the measurement may be obtained from a blood pressure measurement device, such as a blood pressure measuring cuff. The cuff may be coupled to a system running the method. The method may in some examples comprise controlling the blood pressure measurement device to acquire the measurement, or the method may comprise passively receiving a measurement acquired using the device, with measurement timings externally controlled, by e.g. an operator.

The measurement is associated with a tester who performed the measurement. For example, a tester ID may be received with the measurement value, the tester ID being a unique identifier associated with the tester. The tester may for instance input their tester ID before taking a measurement, so that it can be either temporarily stored with the measurement, or received by the method in combination with the measurement value. In some examples, the tester and tester ID can be detected automatically by for instance recognizing them by means of a camera (facial recognition), the tester scanning an ID badge, a finger print scanner, or an eye (retinal) scan.

After obtaining the measurement, the method comprises comparing 14 the measurement with historical measurements for the subject stored in a database, and determining based on this whether the obtained measurement is an outlier for the patient. There may be a global database in which historical measurement values for each subject, and data associated with each tester are stored. Alternatively, there may be a separate (dedicated) database for subject historical data. From the database, a distribution of the subject's past blood pressure readings can be obtained or derived, and statistical analysis methods used to determine whether the newly obtained measurement is within an expected range of the past distribution, or is within an outlier zone. This will be described in greater detail below.

In some examples, determining whether the measurement is an outlier may comprise determining a probability that the measurement is an outlier. If the probability exceeds a defined probability threshold for instance, it may be determined that the measurement is an outlier.

Optionally, if the measurement is not determined to be an outlier measurement for the subject, the measurement may at this point be stored in a measurement database, comprising blood pressure measurements for the patient. It may be labelled as a true measurement or a normal measurement. Its non-outlier status may be labelled. Any derived probability may also be output as part of this output information. The measurement database may be the same database as the database of historical measurements for the subject, or a different database. For example, there may be an active 'clinical' database of current measurements used to store the new measurement, and which clinicians can use to make medical judgments. The database of past measurements might be separate from this. After generating and storing this output information 20, the method may end, with further steps omitted.

In the case the measurement is determined to be an outlier, however, the method further comprises a step of accessing 16 a database of contextual data for the patient, pertaining to contextual factors which have an effect on blood pressure, and determining based on this any contextual cause of the outlier measurement. The contextual factors may include for example information indicative of one or more of: subject medical record, subject medications, date and time of obtained measurement, subject height, subject weight, subject activity levels, whether subject smokes, subject diet, subject stress levels, subject breathing rate. These factors can affect blood pressure readings independently of any underlying medical cause. Ruling these out as possible causes helps distinguish genuine changes in blood pressure from changes due to circumstance.

Determining contextual causes for the outlier may be based on a known or stored relationship or correlation between each factor and blood pressure measurements. For instance, a known general correlation between activity levels and blood pressure may be accessible to the method. Based on this, and based on the contextual data for the subject acquired from the database, the method can determine whether the subject's activity level would normally be associated with an expected blood pressure elevated by the amount which the subject's blood pressure is elevated by. Activity level is used as one example, but the same principle applies to any other contextual factor.

The database of contextual data for the subject may be acquired for instance via a user interface by means of which a subject or medical personnel can manually input the contextual data. This may include for instance keyboard entry, or speech recognition. The database may be at least partially populated based on a subject's medical record to which the method may have access. A wearable device (e.g. a fitness device) may be used to acquire some of the data, e.g. activity level, heart rate, stress levels. The information might be input to the database just before taking the measurement in some examples. For example, a subject may be asked contextual questions before taking the measurement, for instance by a tester and the answers input through a user interface as contextual information. Alternatively the user may input the answers themselves.

Optionally, if a contextual cause is identified for the outlier, the method may comprise at this stage labelling the data with the identified cause, and storing the measurement, along with the cause label, in a measurement database for the subject. This may be the same measurement database as true measurements that are not outliers are stored in, or a different database. The measurement may be labelled as a false judgment, to avoid it being used to make medical decisions about treatment. Alternatively, responsive to identifying a contextual cause, the method may comprise discarding the measurement (since the changes it represents are assumed not be indicative of an underlying medical status of the subject). After generating and exporting this output information, the method may end, with no further steps performed.

Alternatively to the above, the step of exporting the data may be omitted until the very end of the method.

At least in the case that no contextual cause is identified, the method further comprises accessing historical measurement data for the tester (who took the measurement) in a database, and determining 18 based on this whether the outlier is attributable to the tester. This step may optionally also be performed even in the case that a contextual cause is identified, so that maximal information can be acquired about a measurement. It may help for instance to know that there is both a possible contextual cause for an outlier and a possible tester-related cause, as this makes it more certain that the measurement is not a reliable measurement.

Means for assessing based on the tester historical data whether the tester is attributable to the outlier will be described in greater detail below. In some examples, an average error or deviation in the tester's historical measurements compared to measurements for the same patients taken by other testers may be determined or accessed. This may indicate systematic error in measurement technique. The average error may be compared to the deviation between the current outlier and the subject's past measurements to determine whether the two are comparable.

If the outlier is attributable to the tester, the method may comprise at this point labeling the measurement with the identified tester-related cause and exporting it to a measurement database. This may be the same measurement database as true measurements which are not outliers are stored in, or it may be a separate database for outlier measurements. In other examples, the measurement may be discarded in the case that the tester is found to be the likely cause of the outlier measurement

The method additionally comprises generating output information 20 indicative of an outlier status of the measurement, and any determined contextual and/or tester attributed cause. As noted above, in some examples, this step, or parts of it may be performed earlier in the method. The output information may be a data output, for instance in the form of a data record for exporting to one or more databases. It may comprise the measurement labelled with the determined outlier status and any identified cause. The method may comprise exporting the information to a database or to a local or remote data store. If no clear cause has been identified for an outlier, it may be labelled as a genuine measurement, or it may be labelled simply as having no clear cause for instance.

Optionally the method may communicate the output information to a user output device for presenting the information as a sensory output. This may be a display for displaying the information to a user for instance, or a speaker for communicating the information as an audio message. The method may generate and output a recommendation to re-take the measurement in cases that a contextual or tester-related cause is identified, or in cases where no cause is identified (e.g. to confirm the unusual result).

The steps above make reference to obtaining a single measurement. However, multiple measurements may be obtained and analyzed in other examples.

Processes for determining whether a measurement is an outlier, whether the measurement has a contextual cause, and whether the measurement has a tester-attributable cause will now be described in more detail.

Determining whether the obtained measurement is an outlier for the subject may be achieved using well established outlier detection methods, such as for example density-based techniques (e.g. k-nearest neighbor methods or local outlier factor methods). For example, the database of historical measurements for the subject may comprise or permit derivation of a distribution of measurements for the subject over time. The distribution would typically follow a normal distribution, having a median center, with values distributed about this median center. According to certain examples, the above mentioned statistical techniques can be used to determine whether the obtained measurement lies within a defined expectation zone within this distribution (e.g. within a defined confidence interval of this distribution). The width and placement of the expectation zone may be adjusted, to permit the method to be more or less sensitive to deviant measurements. The expectation zone may be defined for instance as a multiple of standard deviations away from the mean or median value of the distribution (a z-score) If the value lies within the defined expectation zone, then the value may be designated as a non-outlier measurement, and if it falls outside, it may be designated as an outlier measurement.

Determining whether there is a contextual cause based on the database of contextual data may be based for example on one or more known correlations or associations between contextual factors and blood pressure. The method can be made more or less sophisticated.

In simple examples, there may be pre-stored known qualitative relationships between contextual factors and blood pressure, i.e. whether a particular contextual factor is known to increase blood pressure or decrease blood pressure. The contextual assessment step may comprise comparing each of a set of the subject's contextual parameters with the known blood pressure relations and determining based on this whether one or more of the factors is linked with blood pressure change. If so, and e.g. if the observed blood pressure change is the same sign (polarity) as the expected change based on the factor, then it may be determined that the contextual factor is a cause of the outlier.

In more complex examples, there may be stored quantitative relationships or correlations between a number of contextual factors and changes in blood pressure. In one set of examples, the method may compute a deviation of the obtained measurement from the mean or median of the subject's historical measurements (a z-score). The contextual assessment step may then comprise comparing each of a set of quantitative contextual parameters for the subject with the known correlations to determine whether the expected change in blood pressure based on the correlation substantially matches the observed deviation of the measurement from the subject's personal mean. If for example, it is found that the observed deviation is within some defined tolerance threshold (which may be pre-set or user-configurable) of the expected blood pressure deviation based on the subject's contextual parameter, then it may be determined that the outlier is caused by that contextual factor. If it is outside of this threshold, the opposite may be concluded.

This provides a more reliable way of using the subject's contextual data to assess whether there is a context-related cause of the outlier.

Other statistical approaches for performing this step are well-known, and will be immediately apparent to the skilled person. Any suitable alternative approach may be used.

By way of one example, the subject may measure his or her blood pressure, which is found to be an outlier (e.g. too high). A dataset of personal characteristics for the subject is consulted, which by way of example indicate the following information:
- Subject's medical history: Heart failure
- Medication adherence: regular
- Body weight: increased

Based on this, the algorithm of the method may determine that this increase in blood pressure is due to fluid accumulation.

The algorithm may then look for other available contextual parameters which might further confirm this conclusion. By way of example, the following information is found:
- Respiration rate (acquired from Wearable device): Elevated

From this, the method may confirm blood pressure is elevated - because of congestion.

By way of one further example, the subject may measure his or her blood pressure and again it is found to be an outlier (too high).

Again, the algorithm consults a dataset of personal characteristics for the subject. The following information is found:
- Subject's medical history: Heart failure
- Medication adherence: regular
- Body Weight: Normal
- Respiration rate: normal

Based on this information, the algorithm of the method determines that this increase in BP is not due to a physio-pathological change (and hence not indicative of an underlying health issue).

Subsequently, the algorithm may in addition perform a check or verification of one or more properties of the measurement signal to identify any anomalies, which may be indicative of further contextual factors related to error in the measurement process or the recorded data. In particular the algorithm may check for and identify any anomalies indicative of measurement errors, for instance attributable to improper attachment of a measurement sensor (e.g. measurement cuff) or significant noise in the measurement signal. In some cases, the algorithm may access data acquired from a movement or position sensor comprised by the measurement device used to acquire the measurement, or mounted to the subject, for instance an accelerometer. This may provide indication that the measurement sensor or the subject was moving during taking of the measurement, or was adopting improper posture during the measurement which may indicate that the measurement is erroneous or flawed. Hence in either of these cases, this further contextual data related to the measurement process itself can be used to confirm or determine that the measurement has a contextual related cause (and is therefore not indicative of any underlying medical issue).

Determining whether an outlier is attributable to a tester may be performed according to one or more examples as follows.

The obtained measurement is associated with a known tester who took the measurement. For example, a tester ID which uniquely identifies the tester may be obtained in combination with the measurement. Other related information may optionally also be known or obtainable such as the day and time when the measurement was taken, and other relevant information such as how many other measurements the tester took the same day, and/or a time between measurements taken by the tester on the day the measurement was taken. This information may be stored in a database, and retrievable based on the tester ID.

There may also be known a clinical status of the tester, e.g. whether the tester is a health care professional or not. This provides an indication as to how well trained the tester is, and so how likely their measurements are to be accurate.

A dataset of historical measurement data for the tester is accessed and based on this, a determination made as to whether an outlier measurement is attributable to the tester.

According to one approach for instance, the database of historical data for the tester comprises performance data, indicative of a historical accuracy of measurements of the tester. The performance data may indicate for instance a measure of an average error of measurements taken by the tester. A distribution of the tester's error for different measurements may be stored.

An average error of the tester cannot in general be directly obtained, as no definite 'true' measurement for a subject is in general available by which each measurement which is taken by a tester can be compared and assessed. Hence the measure of average error is in general an estimate.

In some cases, the measure of error may be based at least in part on a record (e.g. a count) of occasions on which a tester has performed a measurement which was subsequently repeated. For instance, in some examples the system or method may prompt for a measurement to be repeated in case that the measurement was found to be an outlier attributable to contextual causes, for instance related to conditions under which the measurement was taken (e.g. poor patient posture, patient movement or poorly placed testing device). Where a tester has a record indicating a comparatively large number of repeated measurements (e.g. compared to other testers, or compared to some standard benchmark), this may be an indication of poor tester performance. Hence the average error would be higher.

The measure of average error may comprise an absolute count of the tester's repeated measurements, or may be a percentage indicating a percentage of measurements which were repeated for example. In some cases, the measure may comprise a measure of an average difference between the two (or more) obtained values for each repeated measurement. Other examples for quantifying this parameter will be apparent to the skilled person.

According to one or more further examples, the measure of average error may comprise a measure of an average disparity between:
(a) measurements taken by the tester for different subjects and historical measurements for the same subjects and/or
(b) measurements taken by the tester for different subjects and measurements for the same population of subjects taken by other testers.

In either case, a distribution of the mentioned disparity values may be stored in a database. The measure of average disparity may then be taken as the mean or median of the distribution.

The approach indicated by (a) above is illustrated in Fig. 2(a). This schematically illustrates an example distribution 32 of deviation scores of past measurements for a given tester for various subjects, the deviation scores being a deviation of each of the tester measurements from an expected measurement for the subject in question. The expected measurement may for example be an average measurement for the subject based on historical measurements for the subject.

In a simplest approach, determining whether an outlier measurement is attributable to a tester may comprise simply identifying the average error score for the tester and determining whether this average error exceeds some defined error threshold. If it does, the tester may be designated as a poor performer, and optionally based on this alone the outlier measurement may be attributed to the tester.

Alternatively, determining whether the outlier is attributable to the tester may comprise determining a measure of disparity between the newly obtained (outlier) measurement and the historical measurements for the subject (e.g. from a mean value of historical measurements for the subject), and comparing this with the above mentioned measure of average disparity for the tester. The average disparity may be understood as an 'expected' disparity for the tester. If the deviation of the obtained measurement from the average past measurement for the subject is within some defined tolerance or threshold of said average disparity for the tester, then it may be determined that the outlier is caused by the tester (since the deviation of the measurement matches the degree of the tester's past demonstrated systematic error).

However, if the deviation of the obtained measurement from the average past measurement for the subject is outside of a defined tolerance or threshold from the tester's average measurement disparity (e.g. the deviation is bigger or smaller than would be expected based on the tester's past demonstrated error alone), then it may be determined that the outlier is not caused by the tester.

The approach indicated by (b) above is illustrated in Fig. 2(b). This differs from (a) simply in that the average 'error' of the tester's measurements is assessed not by comparison to an average historical measurement for each patient being tested, but by comparison of the whole set of tester measurements on a population of subjects to the set of measurements taken on the same population of subjects by other testers.

Fig. 2(b) schematically illustrates this, illustrating an example distribution 34 of blood pressure measurements for the tester, and on the same axis an example distribution 36 of blood pressure measurements for the same population of subjects, taken by other testers. It can be seen that in this example there is a clear deviation, Δ, in the median/mean centers of the two distributions. This deviation is taken to be the average error value for the tester, as it indicates a systematic error or bias in the measured values for the tester.

As with approach (a), determining whether an outlier is attributable to the tester may comprise determining whether this average disparity value for the tester exceeds some defined threshold. More preferably, it comprises determining a measure of disparity between the newly obtained measurement and the historical measurements for the subject (e.g. from a mean value of historical measurements for the subject), and comparing this with the above mentioned measure of average disparity for the tester.

Again, if the deviation of the obtained measurement from the average past measurement for the subject is within some defined tolerance or threshold of said average disparity for the tester, then it may be determined that the outlier is caused by the tester (since the deviation of the measurement matches the degree of the tester's past demonstrated systematic error).

According to further examples, determining whether an outlier is attributable to a tester may be based on comparing qualitative characteristics of a tester's past errors with characteristics of the detected outlier measurement. For instance if the measurement is determined to be an outlier which exceeds (i.e. falls outside and above) an expected range of values for a subject, but the tester has a history of underestimating measurements, it may be determined that the outlier is not caused by tester error, and vice versa.

This may be based on confidence intervals. For example, a measurement may be determined to be an outlier by virtue of exceeding (i.e. falling outside and above) a predefined confidence interval of the subject's distribution of past measurements. In this case, the tester's historical data may be consulted. If the tester's historical data indicates that they on average underestimate measurements, then this may be an indication that the outlier is not attributable to the tester. If the tester's historical data indicates that they on average overestimate measurements, this may provide an indication that the outlier is attributable to the tester. In some cases this approach may be combined with one or both of the quantitative approaches (a) and (b) outlined above to determine attribution of the outlier with greater certainty.

By way of one example, determining whether the outlier is attributable to the tester may be performed as follows. The tester measures the blood pressure of the subject and it is determined that the measurement is an outlier for the subject based on historical measurement data for the tester. A determination is made that there is no contextual cause of the outlier result. The method next optionally checks a set of stored characteristics or contextual data related to the tester which may influence accuracy of tester measurements. The following information is found:
- Health Care Professional: yes
- Professional Experience: high
- Measurement Conditions: Time pressure
- Movement during the measurement: high

The database of historical measurement data for the tester is then accessed, and an assessment made as to an average error (disparity score) of the tester's measurements. It is found that an outlier history of the tester (disparity score) is low.

The algorithm based on the above information makes a determination that the measurement was taken in a stressful situation and generates output information indicative of this, and optionally also indicating advice to retake the measurement.

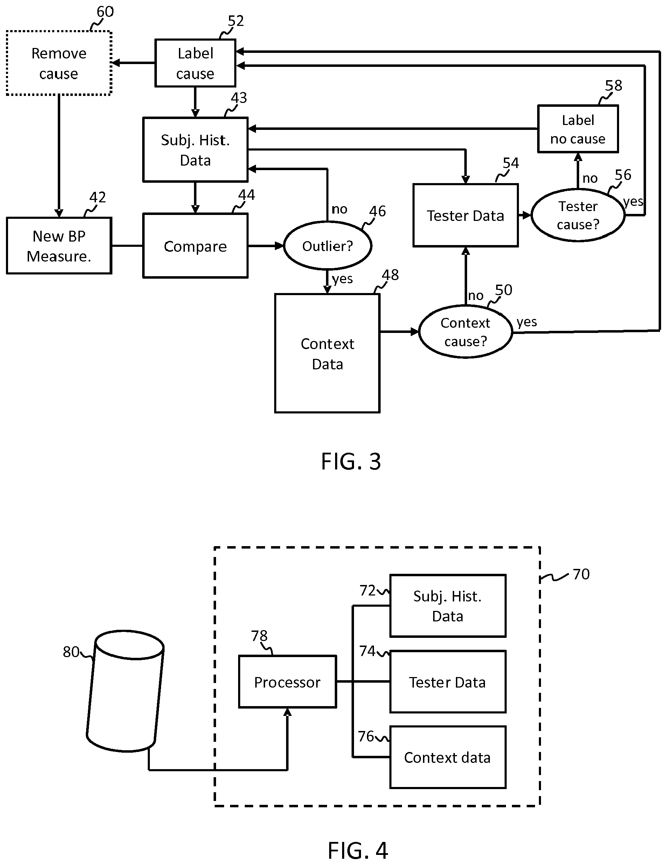

To illustrate the method according the invention in its totality, the complete workflow of one example analysis method according to one or more embodiments will now be described with reference to Fig. 3.

The method starts with obtaining 42 a new blood pressure measurement. A statistical evaluation 44 is then performed of the new measurement based on comparing the measurement to a database 43 of historical measurement data for the subject. The database 43 may be a global database which includes both patient historical measurement data and historical data pertaining to different testers.

Based on the statistical analysis (comparison step) 44, a determination 46 is made as to whether the measurement is an outlier for the patient. If the measurement is not an outlier it is added to the measurement database 43 for the subject. It may optionally be labelled as a true or reliable measurement.

If it is determined 46 that the measurement is an outlier for the patient, a database of contextual data 48 for the subject is accessed, pertaining to contextual factors which have an effect on blood pressure, and based on this a determination 50 is made as to whether there is any contextual cause of the outlier measurement. As noted above, the contextual data may include by way of example only activity levels of the subject, smoking history, diet for the subject and stress levels. Data from a wearable device might also be included in the database or accessed as part of the determination 50 step. A wearable device may for instance provide information on heart rate, activity levels, sleep patterns or any other information which can be collected using a wearable device, e.g. a fitness tracker.

If a contextual cause 50 is identified, the measurement is (according to the present example) labelled 52 with the identified contextual cause and added to the database 43 of measurements for the subject, along with the label. It additionally may be labelled or tagged as unreliable in some examples, so that clinical decisions are not based on it.

Optionally, a further step may also be performed in which an output message is generated 60 advising a user to retake 42 the measurement, due to its outlier status and contextual cause. The cause of the outlier may also be communicated as part of this message.

If a determination is made 50 that there is no contextual cause, a database 54 of historical measurement data for the tester is accessed, and based on this a determination made 56 as to whether the outlier is attributable to the tester (whether the tester is the cause of the outlier). The tester data may optionally be stored in a common global database along with the historical measurement data 43 for the various subjects. The determination is based on a statistical evaluation of the measurement with reference to statistics for the tester, where this may be performance data for the tester. Example methods and procedures for determining any tester-attributable cause of an outlier have been described in detail above.

If it is determined that the outlier is attributable to the tester, the measurement may be labelled 52 with the identified cause and then stored in the measurement database 43 of measurements for the subject, along with the label. It additionally may be labelled or tagged as unreliable in some examples, so that clinical decisions are not based on it.

Again, optionally, a further step may also be performed in which an output message is generated 60 advising a user to retake 42 the measurement, due to its outlier status and tester-attributable cause. The cause of the outlier may also be communicated as part of this message.

If it is determined that there is no tester-attributable cause of the outlier, it is labelled 58 as having no context and no tester-attributable cause. It may be labelled as having an unclear cause. It may be labelled as a true or genuine measurement, so that it can be used in making further clinical decisions regarding treatment for the patient. A warning message may be issued indicating to a user that the measurement may be indicative of a genuine blood pressure change, so that any necessary responsive action can be taken rapidly. The labelled measurement is then stored in the database 43 of historical measurements for the subject, along with its label of no identifiable cause.

The above described method may be implemented by a computer. Hence, a further aspect of the invention provides a computer program comprising code means for implementing the method according to any of the examples or embodiments described above or defined in any claim when said program is run on a computer. For example, the method may be implemented by an app installed on a mobile computing device such as a smartphone or tablet. The mobile device may comprise a display for displaying the output information generated as part of the method.

The method described above maybe implemented by an analysis system, which may optionally comprise means for measuring blood pressure. This aspect of the invention will now be described.

Accordingly, a further aspect of the invention provides a system for analyzing blood pressure measurements. An example is schematically shown in block diagram form in Fig. 4.

The system 70 comprises a first dataset 72 storing historical blood pressure measurements for one or more subjects, a second dataset 74 comprising historical measurement data relating to historical measurements performed by one or more testers. A further dataset 76 is also included comprising contextual data for the subject, pertaining to contextual factors which have an effect on blood pressure.

The system further comprises a processor 78, having access to said datasets, and adapted to:
obtain a blood pressure measurement for a subject, the measurement being associated with a known tester who performed the measurement,
compare the measurement with historical measurements for the subject stored in said first dataset 72, and determining based on this whether the obtained measurement is an outlier for the subject,
in the case the measurement is an outlier, access the further dataset 76 of contextual data for the subject, pertaining to contextual factors which have an effect on blood pressure, and determine based on this any contextual cause of the outlier measurement,
at least in the case of no contextual cause, access historical measurement data for the tester from said second dataset 74, and determine based on this whether the outlier is attributable to the tester; and
generate output information indicative of an outlier status of the measurement, and any determined contextual and/or tester attributed cause.

Optionally, the system 70 further comprises a blood pressure sensing means 80, communicatively coupled to the processor 78, for providing said blood pressure measurement to the processor. Hence in this case the system effectively provides a complete blood pressure measurement system, including components for both measuring blood pressure and analyzing the measurements.

In other examples, the processor may be communicably connectable with a blood pressure sensing means 80 for obtaining the blood pressure measurements. This configuration is illustrated schematically in Fig. 4 for example.

Alternatively still the processor 78 may obtain the blood pressure measurement from a different source, for example from a data store in which the measurement has been temporarily stored upon collection for later analysis by the analysis system 70. The measurement may be obtained from a patient monitor. The blood pressure measurement may alternatively be input manually be a user. The measurement may also be obtained in any other way as will be apparent to the skilled person.

Each of the first 72, second 74 and further 76 datasets may be or may be comprised by respective databases. Each may be part of a separate database or the three datasets may be comprised within a single database. In other examples, the first and second datasets may be comprised by a common database, and the further dataset comprised by a separate database.

The datasets and/or databases comprising the datasets may be stored on a local memory in examples. The memory may be part of the processor 78. In this case or in any case, the datasets may be comprised by the processor 78. Hence although they are shown schematically as separate components in Fig. 4, the actual distribution of these elements may differ.

Although in the example of Fig. 4, the further dataset is comprised by the system 70, in further examples, the further dataset may be external to the system, and wherein the processor 78 is configured to have access to the dataset. The dataset may be stored on a remote server or data store for example, with which the processor is communicable.

The blood pressure measurement means 80 may be a blood pressure sensing device, such as blood pressure cuff. The device may have an integrated processor to generate a measurement of blood pressure based on internal sensor readings, and adapted to generate an output indicative of the derived measurement. The measurement means may be operatively or communicatively coupled to the processor 78 for supplying the obtained blood pressure measurement(s) to the processor.

The method and system of the invention may be applied for analyzing blood pressure measurements either in a clinical e.g. hospital environment, or in a home environment. Hence, the invention is useful both for hospital blood pressure monitoring applications and home-monitoring applications, in which blood pressure of a patient is monitored over an extended period when a patient is at home.

As discussed above, the system makes use of a processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A blood pressure measurement analysis method (10), comprising:
obtaining (12) a blood pressure measurement for a subject, the measurement being associated with a known tester who performed the measurement;
comparing (14) the measurement with historical measurements for the subject stored in a database, and determining based on this whether the obtained measurement is an outlier for the subject;
in the case the measurement is an outlier, accessing (16) a database of contextual data for the subject, pertaining to contextual factors which have an effect on blood pressure, and determining based on this any contextual cause of the outlier measurement;
at least in the case of no contextual cause, accessing (18) historical measurement data for the tester in a database, and determining based on this whether the outlier is attributable to the tester;
generating (20) output information indicative of an outlier status of the measurement, and any determined contextual and/or tester attributed cause.

2. A method (10) as claimed in claim 1, wherein in the event that no contextual or tester-attributable cause is identified for an outlier measurement, the measurement is exported to a measurement database, and labeled as a true measurement.

3. A method (10) as claimed in claim 1 or 2, wherein, in the event that a contextual or tester-attributable cause is identified for an outlier measurement:
the measurement is exported to a measurement database, labelled with the cause, or
the measurement is discarded.

4. A method (10) as claimed in any of claims 1-3, wherein accessing said historical measurement data for the tester is only performed in the case that no contextual cause is identified for the outlier.

5. A method (10) as claimed in any of claims 1-4, wherein the historical measurement data for the tester comprises performance data, indicative of a historical accuracy of measurements of the tester.

6. A method (10) as claimed in claim 5, wherein the performance data indicates a measure of an average error of measurements taken by the tester.

7. A method (10) as claimed in claim 6, wherein the measure of average error is a measure of an average disparity between
measurements taken by the tester for different subjects and measurements for the same subjects taken by other testers, and/or
measurements taken by the tester for different subjects and historical measurements for the same subjects.

8. A method (10) as claimed in claim 6 or 7, wherein determining (18) whether the outlier is attributable to the tester comprises determining a measure of disparity between the obtained measurement and the historical measurements for the subject, and comparing this with said measure(s) of average error for the tester.

9. A method (10) as claimed in any of claims 1-8, wherein determining (14) whether the obtained measurement is an outlier comprises determining a measure of disparity between the obtained measurements and the historic measurements for the subject and determining whether this exceeds a defined disparity threshold.

10. A method (10) as claimed in any of claims 1-9, wherein the contextual information includes information indicative of one or more of: subject medical record, subject medications, date and time of obtained measurement, subject height, subject weight, subject activity levels, whether subject smokes, subject diet, subject stress levels, subject breathing rate.

11. A method (10) as claimed in any of claims 1-10, wherein the method comprises determining a probability that the obtained measurement is an outlier.

12. A computer program comprising code means for implementing the method of any one of claims 1 to 11 when said program is run on a computer.

13. An analysis system (70), comprising:
a first dataset (72) storing historical blood pressure measurements for one or more subjects;
a second dataset (74) comprising historical measurement data relating to historical measurements performed by one or more testers; and
a processor, having access to said datasets, and adapted to:
obtain a blood pressure measurement for a subject, the measurement being associated with a known tester who performed the measurement,
compare the measurement with historical measurements for the subject stored in said first dataset, and determining based on this whether the obtained measurement is an outlier for the subject,
in the case the measurement is an outlier, access a further dataset (76) of contextual data for the subject, pertaining to contextual factors which have an effect on blood pressure, and determine based on this any contextual cause of the outlier measurement,
at least in the case of no contextual cause, access historical measurement data for the tester from said second dataset, and determine based on this whether the outlier is attributable to the tester; and
generate output information indicative of an outlier status of the measurement, and any determined contextual and/or tester attributed cause.

14. A system (70) as claimed in claim 13, further comprising a blood pressure sensing means (80), communicatively coupled to the processor, for providing said blood pressure measurement to the processor.
